# EUROPEAN PATENT SPECIFICATION

(11) **EP 2 925 410 B1**
(45) Date of publication and mention of the grant of the patent: **16.11.2016**
(21) Application number: 12798542.2
(22) Date of filing: 27.11.2012
(51) Int. Cl.: A61Q 11/00, A61K 8/73, A61K 8/81, A61K 8/02, A61K 8/29

(54) **DENTIFRICE COMPOSITIONS CHANGING OF COLOR WHEN TOOTH BRUSHING**
ZAHNPASTE MIT BÜRSTZEITANZEIGER
DENTIFRICE CHANGEANT DE COULEUR LORS DU BROSSAGE DES DENTS

(43) Date of publication of application: 07.10.2015
(73) Proprietor: Colgate-Palmolive Company, New York, NY 10022 (US)
(72) Inventor: PAN, Guisheng, Philadelphia, Pennsylvania 19114 (US); PATEL, Dipak, Parsippany, New Jersey 07054 (US); SZEWCZYK, Gregory, Flemington, New Jersey 08822 (US); LIN, Nora C., Basking Ridge, New Jersey 07920 (US)
(74) Representative: Jenkins, Peter David
(86) International application number: PCT/US2012/066662
(87) International publication number: WO 2014/084813

(56) References cited:
- EP-A1- 0 298 839
- WO-A1-2005/058265
- WO-A1-2005/094767
- US-B1- 6 669 929
- DATABASE WPI Week 200310 Thomson Scientific, London, GB; AN 2003-108754 XP002713036, & KR 2002 0056224 A (CJ CORP) 10 July 2002 (2002-07-10)
- DATABASE CA, [Online] 28 January 1985 (1985-01-28), "Toothpastes containing color indicators for timing adequate brushing", XP002683277, retrieved from CA Database accession no. 102-172447
- DATABASE CA, [Online] 1 January 1985 (1985-01-01), "Toothpastes containing color indicators for timing adequate brushing", XP002683278, retrieved from CA Database accession no. 102-172448

## Description

### BACKGROUND

The present invention relates to orally acceptable films, to a method of making such films, and to dentifrice compositions including such films.

It is recommended that children should brush their teeth for at least 45-60 seconds, and adults for at least 90 to 120 seconds. Most people, especially children, do not brush their teeth for a sufficient period of time to obtain maximum benefit, and moreover have difficulty accurately estimating the time necessary to brush the teeth.

It is known to provide color changing films in toothpaste which release a pigment after a given brushing period. In particular, it is known to provide dark, even black, color changing films within toothpaste which release the dark pigment from the film upon brushing because the brushing action in the oral cavity causes dissolution of the film matrix and release of the dark pigment. Such dark films are inexpensive and have very strong color change signal (high color change contrast). However, such dark films are not aesthetically acceptable to the consumer because the consumer does not want to see dark particles in the toothpaste.

It is also known to provide a color changing film which comprises triple layer laminated sandwich structure of a white film-black film-white film. The white films may incorporate titanium dioxide, TiO₂, in a film matrix similar to the matrix of the central dark film. The outermost white films partially mask or hide the black film in the middle of the sandwich, although the peripheral edge of the black film is not covered by the white films. These triple layer films are more consumer-acceptable visually. However, the color change contrast is diminished due to the very high color-covering power of TiO₂ from the outer layers, and the films are very expensive to make.

There is a need for improved, consumer-friendly products and methods to encourage adults and children to brush their teeth for a longer period of time.

It is an aim of the present invention to provide a color changing film, and a dentifrice including such a film, which can provide a color change signal to increase the brushing time for the consumer by controlling the brushing time at 45-60 sec for children and 90-120 sec for the adults, with a film which can be inexpensive to manufacture, is visually acceptable to the consumer and can provide a strong color change signal.

### SUMMARY

Some embodiments of the present invention provide an orally acceptable film, wherein the film comprises a film substrate comprising a water-soluble polymer matrix which includes a first pigment that is released upon dissolution of the matrix, a second pigment which at least partially masks the at least one surface of the film substrate, and a polymeric material adhering the second pigment to the at least one surface of the film substrate.

In some embodiments, the film is present in the form of dissolvable film fragments coated with second pigment. In some embodiments, the film fragments are formed prior to being coated with the second pigment; in another embodiment the film fragments are formed after being coated with the second pigment.

Some embodiments of the present invention provide a method of making an orally acceptable film, the method comprising the steps of: (a) forming a film substrate comprising a water-soluble polymer matrix which includes a first pigment that is released upon dissolution of the matrix; and either b1. applying a polymeric material to at least one surface of the film substrate; c1. applying a second pigment to the polymeric material which at least partially masks the at least one surface of the film substrate; and d1. adhering the second pigment to the at least one surface of the film substrate by the polymeric material; or b2. applying a polymeric material to a second pigment to form a second pigment coated with polymeric material; c2. applying the second pigment coated with polymeric material to the substrate to at least partially cover at least one surface of the film substrate; and d2. adhering the second pigment coated with polymeric material to the substrate which is at least partially covered on at least one surface of the film substrate.

Some embodiments of the present invention provide a dentifrice including a plurality of film fragments of the orally acceptable film according to the invention or made by the method of the invention. In some embodiments, the dentifrice comprises dissolvable film fragments coated with second pigment, e.g., wherein the film dissolves and releases the first pigment after sufficient brushing.

Other embodiments further provide methods of cleaning the teeth comprising brushing with such a dentifrice until the first pigment is released.

The film and dentifrice compositions of the invention are intended for topical use in the mouth, thus components for use in the present invention should be orally acceptable, that is, safe for topical use in the mouth, in the amounts and concentrations provided.

The inventors have found, in particular embodiments, that by adhering, using a polymeric material as an adhesion agent, a masking (second) pigment onto the film substrate , the relatively dark color changing pigment can be masked by the relatively light color masking pigment until the color changing pigment is released during use to indicate adequate brushing time. The resultant film and dentifrice are aesthetically acceptable to the consumer, the films are inexpensive to manufacture, the masking can be substantially complete and the color change indication is strong.

Furthermore, the polymeric material acting as an adhesion agent ensures a strong bond between the masking pigment and the film, with the result that the masking pigment is not inadvertently abraded from the film surface, so that the relatively dark colored film is not unmasked until the color change indication is released at the desired moment during brushing.

The brushing effect in the aqueous environment within the oral cavity causes dissolution of the polymeric material, which, enhanced by the mechanical rubbing of the film fragments by the brush filaments and against the oral surfaces, causes removal of the masking pigment from the film. The film then disintegrates and dissolves to release the color changing pigment.

This use of a masking (second) pigment adhered to the film substrate can provide the advantage of avoiding the cost and complexity of creating additional masking film layers laminated to the core color changing film.

Also, the entire surface of the film, including the peripheral edge of the film, can advantageously be coated with the masking pigment, whereas in contrast in a multilayer film laminate the outer layers only cover the major surfaces of the central core layer and the peripheral edge of the central core layer is left visually exposed.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

It should be understood that the detailed description and specific examples, while indicating embodiments of the invention, are intended for purposes of illustration only and are not intended to limit the scope of the invention.

The invention thus provides, in a first aspect, an orally acceptable film, wherein the film comprises a film substrate comprising a water-soluble polymer matrix which includes a first pigment that is released upon dissolution of the matrix, a second pigment which at least partially masks the at least one surface of the film substrate, and a polymeric material adhering the second pigment to the at least one surface of the film substrate.

Optionally, the first pigment is released upon dissolution of the film and may comprise a pigment or combination of pigments, e.g., selected from a red pigment, for example D&C Red 30, a green pigment, for example Pigment Green 7, a yellow pigment, e.g. (Natpure LC 128 Yellow, from Sensient Co.), or a blue pigment, for example a phthalocyanine, for example Pigment Blue 15:

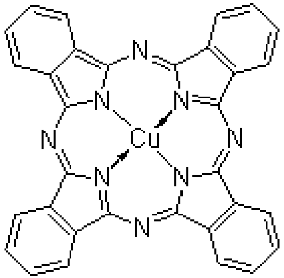

In some embodiments, the first pigment in the film matrix comprises a polar pigment.

Optionally, the polymeric material is water-soluble. Typically, the polymeric material comprises polyvinylpyrrolidone.

In some embodiments, the second pigment is in the form of powder particles. The second pigment may be a white powder pigment, e.g., comprising particles of titanium dioxide or Ca₂P₂O₇, or alternatively a red powder pigment, e.g., comprising particles of red iron oxide or D&C Red 30. Typically, the second pigment comprises titanium dioxide and the masked coated films appear white in color.

The second pigment may optionally comprise a polar compound and/or a water-insoluble material, for example a water-insoluble antimicrobial agent, e.g., selected from triclosan and zinc oxide.

In some embodiments, the polymeric material comprises from 5 to 25 wt% of the total weight of the second pigment and the polymeric material, further optionally from 10 to 20 wt% of the total weight of the second pigment and the polymeric material.

In some embodiments, the second pigment comprises from 5 to 25 wt% of the weight of the film substrate, further optionally from 10 to 20 wt% of the weight of the film substrate.

Typically, the second pigment comprises titanium dioxide powder which comprises from 5 to 17 wt% of the weight of the film substrate and the polymeric material comprises polyvinylpyrrolidone in an amount of from 5 to 17 wt% of the total weight of the titanium dioxide powder and the polyvinylpyrrolidone.

In some embodiments, the water soluble polymer matrix comprises cellulose ethers, e.g., selected from (i) alkylcellulose, e.g., methylcellulose; (ii) hydroxyalkyl cellulose, e.g., selected from hydroxypropyl methyl cellulose, hydroxyethyl propyl cellulose, hydroxybutyl methyl cellulose, hydroxypropyl methyl cellulose, carboxymethyl cellulose and mixtures thereof; and (iii) mixtures thereof.

Typically, the matrix further comprises at least one of a starch, a plasticizer and a non-ionic surfactant or emulsifier, or any combination or two or more of these components. The starch may be a pre-gelatinized starch. The plasticizer may be a polyalcohol, e.g., sorbitol, propylene glycol, glycerol, or low molecular weight polyethylene glycol, e.g., PEG 200. For example, the film matrix may comprise propylene glycol, e.g., in an amount effective to provide plasticity to the film, e.g., about 20-30 wt% by dry weight of the film. In some embodiments, the non-ionic surfactant or emulsifier is a polysorbate. The polysorbate may be polysorbate 80 (also known as polyoxyethylene (20) sorbitan monooleate, available commercially e.g., as Tween® 80), e.g., in an amount of about 1-5 wt% by dry weight of the film.

Optionally, the film matrix comprises, by dry weight of the matrix, (i) 20-60 wt% cellulose ethers selected from methyl cellulose, hydroxypropylmethyl cellulose, and mixtures thereof, (ii) 10-30 wt% propylene glycol; 1-5 wt% polysorbate 80, and 15-55 wt% first pigment.

Typically, the film substrate is present in the form of film fragments. Optionally, the film fragments have a maximum dimension of 4mm, for example from 1 to 4 mm. The film may have an average thickness of from about 0.025 to about 0.075mm (1 to 3 thousandths of an inch). The film fragments may have any desired shape and dimensions for aesthetic purposes. For example, the film fragments may be square, or any other geometrical or non-geometrical shape.

In some embodiments, the second pigment covers substantially the entire surface of each film fragment. For example, the entire surface of each film fragment includes two opposed faces and a peripheral edge surface therebetween.

In some embodiments, the orally acceptable film is suspended in a dentifrice and the first and second pigments are insoluble during brushing of teeth using the dentifrice in the presence of water within an oral cavity.

In some embodiments, the orally acceptable film is suspended in a dentifrice and the matrix is substantially dissolved after a period of greater than 30 seconds and less than 180 seconds of brushing of teeth using the dentifrice in the presence of water within an oral cavity.

The invention also provides, in a second aspect, a method of making an orally acceptable film, the method comprising the steps of:
a. forming a film substrate comprising a water-soluble polymer matrix which includes a first pigment that is released upon dissolution of the matrix; and either
b1. applying a polymeric material to at least one surface of the film substrate;
c1. applying a second pigment to the polymeric material which at least partially covers the at least one surface of the film substrate; and
d1. adhering the second pigment to the at least one surface of the film substrate by the polymeric material; or
b2. applying a polymeric material to a second pigment to form a second pigment coated with polymeric material;
c2. applying the second pigment coated with polymeric material to the substrate to at least partially cover at least one surface of the film substrate; and
d2. adhering the second pigment coated with polymeric material to the substrate which is at least partially covered on at least one surface of the film substrate.

In some embodiments, composition of any or all of the matrix, the first pigment, the second pigment and the polymeric material may be as discussed above for the first aspect of the invention.

In some embodiments, in steps b1 or b2 the polymeric material is applied in the form of a liquid. Optionally, in steps b1 or b2 the polymeric material is sprayed as a liquid onto the at least one surface of the film substrate or the second pigment.

Typically, in step b1 the polymeric material is in solution in a solvent and in step d1 the solvent is evaporated to leave a layer of polymeric material adhering the second pigment to the at least one surface of the film substrate; or alternatively, in step b2 the polymeric material is in solution in a solvent and the solvent is evaporated to form a second pigment coated with polymeric material which is then adhered to the at least one surface of the film substrate.

In some embodiments, the polymeric material comprises polyvinylpyrrolidone. In some embodiments, in step b1 the polyvinylpyrrolidone is applied as a solution in a non-aqueous solvent which is evaporated in step d1. Typically, the solvent is ethanol. In some embodiments, in step b2 the polyvinylpyrrolidone is applied as a solution in a non-aqueous solvent which is evaporated to form a second pigment coated with polymeric material. Typically, the solvent is ethanol.

In some embodiments, the polyvinylpyrrolidone is dissolved in the solvent in an amount of from 10 to 30 wt% based on the total weight of the polyvinylpyrrolidone and the solvent, further optionally from 15 to 25 wt% based on the total weight of the polyvinylpyrrolidone and the solvent.

In some embodiments, wherein in step d the coated film substrate is heated. Typically, in step d the heating is carried out for a period of from 30 to 90 minutes at a temperature of from 75 to 125 °C.

In some embodiments, in step c1 or b2 the second pigment is in the form of powder particles. For example, the second pigment may comprise titanium dioxide.

In some embodiments, the polymeric material comprises from 5 to 25 wt% of the total weight of the second pigment coated with polymeric material. In another embodiment, the polymeric material comprises from 10 to 20 wt% of the total weight of the second pigment coated with the polymeric material.

In some embodiments, the second pigment comprises from 5 to 25 wt% of the weight of the film substrate, further optionally from 10 to 20 wt% of the weight of the film substrate.

In some embodiments, the second pigment comprises titanium dioxide powder in an amount of from 5 to 17 wt% of the film substrate, and the polymeric material comprises polyvinylpyrrolidone in an amount of from 5 to 17 wt% of the total weight of the titanium dioxide powder and the polyvinylpyrrolidone.

Typically, the film substrate is present in the form of film fragments. The film fragments may optionally have the dimensions and shape as discussed above for the first aspect of the invention.

In some embodiments, in step b the polymeric material is applied so as to cover substantially the entire surface of each film fragment and in step c the powder coating is applied so as to cover substantially the entire surface of each film fragment. Optionally, the entire surface of each film fragment includes two opposed faces and a peripheral edge surface therebetween.

The invention further provides, in a third aspect, a dentifrice including a plurality of film fragments of the orally acceptable film according to any embodiments of the invention or made by the method according to any embodiments of the invention.

In some embodiments, the dentifrice comprises from 0.3 to 1 wt% of film fragments based on the weight of the dentifrice.

In some embodiments, the dentifrice is a translucent or transparent gel, for example a clear gel toothpaste, having the plurality of film fragments substantially uniformly suspended therein.

In some embodiments, the first pigment is invisible to the naked eye and the second pigment is visible to the naked eye prior to brushing of teeth using the dentifrice in the presence of water within an oral cavity.

In some embodiments, the first pigment is visible to the naked eye and the second pigment is visible to the naked eye after the matrix is substantially dissolved after a period of brushing of teeth using the dentifrice in the presence of water within an oral cavity.

In some embodiments, upon use of the dentifrice or toothpaste, the film matrix substantially dissolves after a brushing period which is typically at least about 30 seconds to about 180 seconds or alternatively from at least about 45 seconds to about 150 seconds. In another embodiment of the invention, the brushing period is selected from a range consisting of about 30 to about 75 seconds, about 45 seconds to about 60 seconds and about 90 seconds to about 120 seconds. In still another embodiment of the invention, the brushing period is from about 45-60 seconds in a toothpaste for use by a child and about 90-120 seconds in a toothpaste for use by an adult. The cited brushing periods should release the first pigment and provide a color signal to the user of adequate brushing.

For example, in one embodiment, the toothpaste is a clear gel, in which the film fragments can be seen clearly. The film fragments may be, for example, small squares 1 to 4 mm across. They may be all one color or assorted colors, the color being imparted by the second pigment, and the film matrix may contain a high concentration of first pigment. After a period of brushing, e.g., at least 30 seconds, the film matrix is disrupted, and the clear gel toothpaste is suddenly colored by the first pigment, signaling to the user that he or she has brushed for an adequate period.

The invention further provides a method of cleaning the teeth comprising brushing with a toothpaste comprising an orally acceptable dissolvable film, e.g. as described in the preceding paragraph, wherein the dissolvable film comprises a first pigment in the film matrix and brushing is continued until the film matrix dissolves and the first pigment provides a color signal to the user of adequate brushing, for example when the brushing time before the film matrix dissolves is between 30 and 180 seconds, e.g., about 45-60 seconds for a toothpaste for use by a child and about 90-120 seconds for a toothpaste for use by an adult.

As used throughout, ranges are used as shorthand for describing each and every value that is within the range. Any value within the range can be selected as the terminus of the range. Unless otherwise specified, all percentages and amounts expressed herein and elsewhere in the specification should be understood to refer to percentages by weight.

The following examples further describe and demonstrate illustrative embodiments within the scope of the present invention. The examples are given solely for illustration and are not to be construed as limitations of this invention as many variations are possible without departing from the spirit and scope thereof. Various modifications of the invention in addition to those shown and described herein should be apparent to those skilled in the art and are intended to fall within the appended claims.

### EXAMPLE

### Example 1

A film was made by producing a water-based slurry having the composition indicated in Table 1. The slurry was cast onto a low energy surface composed of a TEFLON® sheet coated with a release agent to facilitate permit separation of the resultant film from the surface. The slurry was then dried to obtain a film having an average thickness of about 0.025 mm (1 thousandth of an inch, or 1 mil). The composition of the dry film is also indicated in Table 1.

**Table 1**

| Ingredient | Weight % of slurry | Weight % of dry film |
|---|---|---|
| Water | 66.8 | 0 |
| Hydroxypropylmethyl cellulose (Methocel E5) | 7 | 21.1 |
| Pigment (Blue 15) | 17 | 51.2 |
| Propylene Glycol | 8 | 24.1 |
| Polysorbate (Tween 80) | 1.2 | 3.6 |
| Total Amount | 100 | 100 |

The resultant film appeared black, due to the high pigment concentration. The pigment, Pigment Blue 15, is encapsulated into the film to trigger color change during brushing when the film is incorporated into a dentifrice.

The black film was cut into film fragments, which were square in shape and had dimensions of 1.6 mm x 1.6 mm.

The film fragments were mixed with a solution of a polymeric material, polyvinylpyrrolidone (PVP), in a non-aqueous solvent in order to coat the entire surface of the film fragments in the polymeric material. In particular, the solution comprised 20 wt% PVP in ethanol. In order to ensure effective and uniform coating of the polymeric material onto the surface of the film fragments, 40 g of the film fragments were mixed with 6 g of the 20 wt% PVP/ethanol solution. In other words, the PVP/ethanol solution comprised 15 wt% of the weight of the film fragments.

In this example, the mixing was achieved by a tumbling machine or by shaking the mixture by hand but in other examples the solution of a polymeric material is applied to the film fragments by spraying of the liquid solution under pressure onto the film fragments while mixing the fragments.

Thereafter, 6 g of titanium dioxide powder was added to the mixture, and the mixture was mixed until the film fragments were uniformly coated with the of titanium dioxide powder particles.

The amount of titanium dioxide powder was selected in order to ensure effective and uniform coating of the titanium dioxide powder onto the surface of the film fragments, so that the black color of the film fragments was substantially entirely masked, on all exposed surfaces of the fragments, not only the major faces but also the peripheral edge, by the white color of the titanium dioxide powder.

The coated fragments appeared white to the naked eye.

In this example, the titanium dioxide powder comprised 15 wt% of the weight of the film fragments.

The coated film fragments were then laid onto a bed and heated at a temperature of 100 °C for a period of 1 hour. This heating step caused the ethanol solvent to evaporate, resulting in uniformly white colored dry film fragments.

The PVP polymeric material coating on the dried film fragments acted to adhere the titanium dioxide powder coating onto the surface of the film fragments, so that the black color of the film fragments was substantially entirely masked, on all exposed surfaces of the fragments, and the dried film fragments appeared white to the naked eye.

The white coated film fragments were incorporated into a clear gel toothpaste which was colored green. The film fragments comprised 0.3 wt% of the total weight of the resultant toothpaste. In the toothpaste, the white coated film fragments could be seen with the naked eye and appeared uniformly white. The film fragments were stable in the gel toothpaste until use.

The toothpaste was used to brush the teeth of a subject. During brushing, the film swelled with water and disintegrated, releasing the blue pigment and, thus, a color change occurred to indicate to the consumer when the brushing was completed sufficiently. After a period of from 90 to 120 seconds, the blue 15 pigment was released from the matrix into the oral cavity to tint the toothpaste blue within the oral cavity. This color change was an indication of sufficient brushing time.

Other modifications and embodiments will be readily apparent to those skilled in the art and are included within the scope of the invention.

## Claims

1. An orally acceptable film, wherein the film comprises a film substrate comprising a water-soluble polymer matrix which includes a first pigment that is released upon dissolution of the matrix, a second pigment which at least partially masks at least one surface of the film substrate, and a polymeric material adhering the second pigment to the at least one surface of the film substrate.

2. The film according to claim 1 wherein:
the polymeric material is water-soluble, and/or
wherein the polymeric material comprises polyvinylpyrrolidone; and/or
the second pigment is in the form of powder particles; and/or
the second pigment comprises titanium dioxide.

3. The film according to any foregoing claim wherein
the matrix comprises a hydroxyalkyl cellulose, preferably hydroxypropyl methyl cellulose; and/or
the matrix further comprises at least one of a starch, a plasticizer, propylene glycol and a non-ionic surfactant or emulsifier, or any combination of two or more of these components, preferably wherein the non-ionic surfactant or emulsifier is a polysorbate.

4. The film according to any foregoing claim which is suspended in a dentifrice and wherein:
the first and second pigments are insoluble during brushing of teeth using the dentifrice in the presence of water within an oral cavity; and/or
wherein the matrix is substantially dissolved after a period of greater than 30 seconds and less than 180 seconds of brushing of teeth using the dentifrice in the presence of water within an oral cavity.

5. A method of making an orally acceptable film, the method comprising the steps of:
a. forming a film substrate comprising a water-soluble polymer matrix which includes a first pigment that is released upon dissolution of the matrix; and either
b1. applying a polymeric material to at least one surface of the film substrate;
c1. applying a second pigment to the polymeric material which at least partially masks the at least one surface of the film substrate; and
d1. adhering the second pigment to the at least one surface of the film substrate by the polymeric material; or
b2. applying a polymeric material to a second pigment to form a second pigment coated with polymeric material;
c2. applying the second pigment coated with polymeric material to the substrate to at least partially cover at least one surface of the film substrate; and
d2. adhering the second pigment coated with polymeric material to the substrate which is at least partially covered on at least one surface of the film substrate.

6. The method according to claim 5 wherein in step b1 or b2 the polymeric material is applied in the form of a liquid, preferably wherein:
in step b1 the polymeric material is sprayed as a liquid onto the at least one surface of the film substrate; and/or
wherein in step b1 the polymeric material is in solution in a solvent and in step dl the solvent is evaporated to leave a layer of polymeric material adhering the second pigment to the at least one surface of the film substrate.

7. The method according to any one of claims 5 or 6 wherein:
the second pigment comprises titanium dioxide; and/or
the polymeric material comprises polyvinylpyrrolidone, preferably wherein in step b1 the polyvinylpyrrolidone is applied as a solution in a non-aqueous solvent,
preferably ethanol, which is evaporated in step d1, more preferably wherein the polyvinylpyrrolidone is dissolved in the solvent in an amount of from 10 to 30 wt%, even more preferably in an amount of from 15 to 25 wt%, based on the total weight of the polyvinylpyrrolidone and the solvent.

8. The method according to any one of claims 5 to 7 wherein:
in step d the coated film substrate is heated, preferably wherein the heating is carried out for a period of from 30 to 90 minutes at a temperature of from 75 to 125 °C; and/or
in step c the second pigment is in the form of powder particles.

9. The film according to any one of claims 1 to 4 or the method according to any one of claims 5 to 8 wherein:
the polymeric material comprises from 5 to 25 wt%, preferably from 10 to 20 wt%, of the total weight of the second pigment and the polymeric material; and/or
the second pigment comprises from 5 to 25 wt%, preferably from 10 to 20 wt%,
of the weight of the film substrate; and/or
the second pigment comprises titanium dioxide powder in an amount of from 5 to 17 wt% of the film substrate, and the polymeric material comprises polyvinylpyrrolidone in an amount of from 5 to 17 wt% of the total weight of the titanium dioxide powder and the polyvinylpyrrolidone.

10. The film according to any one of claims 1 to 4 or the method according to any one of claims 5 to 9 wherein the film substrate is present in the form of film fragments, preferably wherein the film fragments have a maximum dimension of from 1 to 4 mm and a thickness of from 0.025 to 0.075 mm.

11. The film according to claim 10 wherein the second pigment covers substantially the entire surface of each film fragment, preferably wherein the entire surface of each film fragment includes two opposed faces and a peripheral edge surface therebetween.

12. The method of claim 10 wherein:
the film fragments are formed before the second pigment or second pigment coated with polymeric material is applied to the film fragments; and/or
wherein in step b1 the polymeric material is applied so as to cover substantially the entire surface of each film fragment and in step c1 the second pigment is applied so as to cover substantially the entire surface of each film fragment,
preferably wherein the entire surface of each film fragment includes two opposed faces and a peripheral edge surface therebetween.

13. The method of claim 10, wherein the film fragments are formed after the second pigment or second pigment coated with polymeric material is applied to the substrate.

14. A dentifrice including a plurality of film fragments of the orally acceptable film according to any one of claims 1 to 4 or made by the method of any one of claims 5 to 12.

15. The dentifrice of claim 14 comprising from 0.3 to 1 wt% of film fragments based on the weight of the dentifrice, wherein:
the dentifrice is a translucent or transparent gel having the plurality of film fragments substantially uniformly suspended therein; and/or
the first pigment is invisible to the naked eye and the second pigment is visible to the naked eye prior to brushing of teeth using the dentifrice in the presence of water within an oral cavity, preferably wherein the first pigment is visible to the naked eye and the second pigment is visible to the naked eye after the matrix is substantially dissolved after a period of brushing of teeth using the dentifrice in the presence of water within an oral cavity.

## Patentansprüche

1. Oral akzeptabler Film, wobei der Film ein Filmsubstrat umfasst, das eine wasserlösliche Polymermatrix umfasst, die ein erstes Pigment, das bei Lösung der Matrix freigesetzt wird, ein zweites Pigment, das mindestens eine Oberfläche des Filmsubstrats zumindest zum Teil abdeckt, und ein polymeres Material beinhaltet, das das zweite Pigment an die mindestens eine Oberfläche des Filmsubstrats haftet.

2. Film nach Anspruch 1, wobei:
das polymere Material wasserlöslich ist und/oder
wobei das polymere Material Polyvinylpyrrolidon umfasst und/oder
das zweite Pigment in der Form von Pulverteilchen ist und/oder
das zweite Pigment Titandioxid umfasst.

3. Film nach einem vorhergehenden Anspruch, wobei
die Matrix eine Hydroxyalkylcellulose, vorzugsweise Hydroxypropylmethylcellulose umfasst und/oder
die Matrix weiterhin mindestens eine/einen/eines von einer Stärke, einem Weichmacher, Propylenglykol und einem nichtionischen Tensid oder Emulgator oder eine beliebige Kombination von zwei oder mehr dieser Komponenten umfasst, vorzugsweise wobei das nichtionische Tensid oder der nichtionische Emulgator ein Polysorbat ist.

4. Film nach einem vorhergehenden Anspruch, der in einem Zahnputzmittel suspendiert ist und wobei:
das erste und das zweite Pigment während eines Zähneputzens unter Verwendung des Zahnputzmittels in Gegenwart von Wasser in einer Mundhöhle unlöslich sind und/oder wobei die Matrix nach einem Zeitraum von mehr als 30 Sekunden und weniger als 180 Sekunden eines Zähneputzens unter Verwendung des Zahnputzmittels in Gegenwart von Wasser in einer Mundhöhle im Wesentlichen gelöst ist.

5. Verfahren zum Herstellen eines oral akzeptablen Films, wobei das Verfahren die folgenden Schritte umfasst:
a. Bilden eines Filmsubstrats, das eine wasserlösliche Polymermatrix umfasst, die ein erstes Pigment beinhaltet, das bei Lösung der Matrix freigesetzt wird; und entweder b1. Aufbringen eines polymeren Materials auf mindestens eine Oberfläche des Filmsubstrats;
c1. Aufbringen eines zweiten Pigments auf das polymere Material, das die mindestens eine Oberfläche des Filmsubstrats zumindest zum Teil abdeckt; und
d1. Haften des zweiten Pigments an die mindestens eine Oberfläche des Filmsubstrats durch das polymere Material; oder
b2. Aufbringen eines polymeren Materials auf ein zweites Pigment, um ein zweites Pigment zu bilden, das mit polymerem Material beschichtet ist;
c2. Aufbringen des zweiten Pigments, das mit polymerem Material beschichtet ist, auf das Substrat, um mindestens eine Oberfläche des Filmsubstrats zumindest zum Teil zu bedecken; und
d2. Haften des zweiten Pigments, das mit polymerem Material beschichtet ist, an das Substrat, das auf mindestens einer Oberfläche des Filmsubstrats zumindest zum Teil bedeckt ist.

6. Verfahren nach Anspruch 5, wobei in Schritt b1 oder b2 das polymere Material in der Form einer Flüssigkeit aufgebracht wird, vorzugsweise wobei:
in Schritt b1 das polymere Material als eine Flüssigkeit auf die mindestens eine Oberfläche des Filmsubstrats gesprüht wird und/oder wobei in Schritt b1 das polymere Material in Lösung in einem Lösungsmittel ist und in Schritt d1 das Lösungsmittel abgedampft wird,
um eine Schicht aus polymerem Material zu hinterlassen, die das zweite Pigment an die mindestens eine Oberfläche des Filmsubstrats haftet.

7. Verfahren nach einem der Ansprüche 5 oder 6, wobei:
das zweite Pigment Titandioxid umfasst und/oder das polymere Material Polyvinylpyrrolidon umfasst, vorzugsweise wobei in Schritt b1 das Polyvinylpyrrolidon als eine Lösung in einem nichtwässrigen Lösungsmittel, vorzugsweise Ethanol aufgebracht wird, das in Schritt d1 abgedampft wird, mehr bevorzugt wobei das Polyvinylpyrrolidon in dem Lösungsmittel in einer Menge von 10 bis 30 Gew.-%, noch mehr bevorzugt in einer Menge von 15 bis 25 Gew.-%, bezogen auf das Gesamtgewicht des Polyvinylpyrrolidons und des Lösungsmittels, gelöst wird.

8. Verfahren nach einem der Ansprüche 5 bis 7, wobei:
in Schritt d das beschichtete Filmsubstrat erhitzt wird, vorzugsweise wobei das Erhitzen für einen Zeitraum von 30 bis 90 Minuten bei einer Temperatur von 75 bis 125 °C durchgeführt wird; und/oder
in Schritt c das zweite Pigment in der Form von Pulverteilchen ist.

9. Film nach einem der Ansprüche 1 bis 4 oder Verfahren nach einem der Ansprüche 5 bis 8, wobei:
das polymere Material von 5 bis 25 Gew.-%, vorzugsweise von 10 bis 20 Gew.-% des Gesamtgewicht des zweiten Pigments und des polymeren Materials ausmacht und/oder das zweite Pigment von 5 bis 25 Gew.-%,
vorzugsweise von 10 bis 20 Gew.-% des Gewichts des Filmsubstrats ausmacht und/oder das zweite Pigment Titandioxidpulver in einer Menge von 5 bis 17 Gew.-% des Filmsubstrats umfasst und das polymere Material Polyvinylpyrrolidon in einer Menge von 5 bis 17 Gew.-% des Gesamtgewichts des Titandioxidpulvers und des Polyvinylpyrrolidons umfasst.

10. Film nach einem der Ansprüche 1 bis 4 oder Verfahren nach einem der Ansprüche 5 bis 9, wobei das Filmsubstrat in der Form von Filmfragmenten vorliegt, vorzugsweise wobei die Filmfragmente eine Höchstabmessung von 1 bis 4 mm und eine Dicke von 0,025 bis 0,075 mm aufweisen.

11. Film nach Anspruch 10, wobei das zweite Pigment im Wesentlichen die gesamte Oberfläche jedes Filmfragments bedeckt, vorzugsweise wobei die gesamte Oberfläche jedes Filmfragments zwei entgegengesetzte Seiten und eine Umfangskantenoberfläche dazwischen beinhaltet.

12. Verfahren nach Anspruch 10, wobei:
die Filmfragmente gebildet werden, bevor das zweite Pigment oder das zweite Pigment, das mit polymerem Material beschichtet ist, auf die Filmfragmente aufgebracht wird; und/oder wobei in Schritt b1 das polymere Material so aufgebracht wird, dass es im Wesentlichen die gesamte Oberfläche jedes Filmfragments bedeckt, und in Schritt c1 das zweite Pigment so aufgebracht wird, dass es im Wesentlichen die gesamte Oberfläche jedes Filmfragments bedeckt, vorzugsweise wobei die gesamte Oberfläche jedes Filmfragments zwei entgegengesetzte Seiten und eine Umfangskantenoberfläche dazwischen beinhaltet.

13. Verfahren nach Anspruch 10, wobei die Filmfragmente gebildet werden, nachdem das zweite Pigment oder das zweite Pigment, das mit polymerem Material beschichtet ist, auf das Substrat aufgebracht wurde.

14. Zahnputzmittel, das mehrere Filmfragmente des oral akzeptablen Films nach einem der Ansprüche 1 bis 4 oder des mit dem Verfahren nach einem der Ansprüche 5 bis 12 hergestellten oral akzeptablen Films beinhaltet.

15. Zahnputzmittel nach Anspruch 14, das 0,3 bis 1 Gew.-% Filmfragmente umfasst, bezogen auf das Gewicht des Zahnputzmittels, wobei:
das Zahnputzmittel ein durchscheinendes oder transparentes Gel ist, wobei die mehreren Filmfragmente im Wesentlichen gleichmäßig darin suspendiert sind; und/oder
vor einem Zähneputzen unter Verwendung des Zahnputzmittels in Gegenwart von Wasser in einer Mundhöhle das erste Pigment mit bloßem Auge nicht sichtbar ist und das zweite Pigment mit bloßem Auge sichtbar ist, vorzugsweise wobei das erste Pigment mit bloßem Auge sichtbar ist und das zweite Pigment mit bloßem Auge sichtbar ist, nachdem die Matrix nach einem Zeitraum eines Zähneputzens unter Verwendung des Zahnputzmittels in Gegenwart von Wasser in einer Mundhöhle im Wesentlichen gelöst ist.

## Revendications

1. Un film buccalement acceptable, dans lequel le film comprend un substrat de film comprenant une matrice polymère soluble dans l'eau qui inclut un premier pigment qui est libéré lors de la dissolution de la matrice, un deuxième pigment qui masque au moins partiellement une ou plusieurs surfaces du substrat de film, et un matériau polymère faisant adhérer le deuxième pigment à la ou aux surfaces du substrat de film.

2. Le film selon la revendication 1 dans lequel :
le matériau polymère est soluble dans l'eau, et / ou
dans lequel le matériau polymère comprend du polyvinylpyrrolidone, et / ou
le deuxième pigment est sous forme de particules de poudre, et / ou
le deuxième pigment comprend du dioxyde de titane.

3. Le film selon l'une quelconque des revendications précédentes dans lequel :
la matrice comprend une cellulose d'hydroxyalkyle, de préférence de la cellulose d'hydroxypropyle méthyle, et / ou
la matrice comprend en outre au moins un composant parmi un amidon, un plastifiant, du propylène glycol et un tensioactif non ionique ou un émulsifiant, ou une combinaison de deux ou plus de deux de ces composants, de préférence dans lequel le tensioactif non ionique ou l'émulsifiant est un polysorbate.

4. Le film selon l'une quelconque des revendications précédentes qui est en suspension dans un dentifrice et dans lequel :
les premier et deuxième pigments sont insolubles pendant le brossage des dents en utilisant le dentifrice en présence d'eau dans la cavité buccale ; et / ou
dans lequel la matrice est sensiblement dissoute après une durée supérieure à 30 secondes et inférieure à 180 secondes de brossage des dents en utilisant le dentifrice en présence d'eau dans la cavité buccale.

5. Un procédé de fabrication d'un film buccalement acceptable, le procédé comprenant les étapes consistant à :
a. former un substrat de film comprenant une matrice polymère soluble dans l'eau qui inclut un premier pigment qui est libéré lors de la dissolution de la matrice ; et soit :
b1. appliquer un matériau polymère à une ou plusieurs surfaces du substrat de film ;
c1. appliquer un deuxième pigment au matériau polymère qui masque au moins partiellement la ou les surfaces du substrat de film ; et
d1. faire adhérer le deuxième pigment à la ou aux surfaces du substrat de film par le matériau polymère ; ou
b2. appliquer un matériau polymère à un deuxième pigment pour former un deuxième pigment revêtu du matériau polymère ;
c2. appliquer le deuxième pigment revêtu du matériau polymère au substrat pour couvrir au moins partiellement une ou plusieurs surfaces du substrat de film ; et
d2. faire adhérer le deuxième pigment revêtu du matériau polymère au substrat qui est au moins partiellement couvert sur une ou plusieurs surfaces du substrat de film.

6. Le procédé selon la revendication 5 dans lequel, à l'étape b1 ou b2, le matériau polymère est appliqué sous forme de liquide, de préférence dans lequel :
à l'étape b1, le matériau polymère est pulvérisé sous forme liquide sur la ou les surfaces du substrat de film ; et / ou
dans lequel, à l'étape b1, le matériau polymère est en solution dans un solvant et, à l'étape d1, le solvant est évaporé pour laisser une couche de matériau polymère adhérer au deuxième pigment à la ou aux surfaces du substrat de film.

7. Le procédé selon l'une quelconque des revendications 5 ou 6 dans lequel :
le deuxième pigment comprend du dioxyde de titane : et / ou
le matériau polymère comprend du polyvinylpyrrolidone, de préférence dans lequel, à l'étape b1, le polyvinylpyrrolidone est appliqué sous forme de solution dans un solvant non aqueux qui est évaporé à l'étape d1, plus préférablement dans lequel le polyvinylpyrrolidone est dissous dans le solvant dans une quantité comprise entre 10 et 30 % en poids sur la base du poids total du polyvinylpyrrolidone et du solvant, plus préférablement encore dans une quantité comprise entre 15 et 25 % en poids, sur la base du poids total du polyvinylpyrrolidone et du solvant.

8. Le procédé selon l'une quelconque des revendications 5 à 7 dans lequel :
à l'étape d, le substrat de film revêtu est chauffé, de préférence dans lequel, le chauffage est effectué pendant une période comprise entre 30 et 90 minutes à une température comprise entre 75 et 125°C ; et / ou
à l'étape c, le deuxième pigment est sous forme de particules de poudre.

9. Le film selon l'une quelconque des revendications 1 à 4 ou le procédé selon l'une quelconque des revendications 5 à 8 dans lequel :
le matériau polymère comprend entre 5 et 25 % en poids, de préférence entre 10 et 20 % en poids du poids total du deuxième pigment et du matériau polymère ; et / ou le deuxième pigment comprend entre 5 et 25 % en poids, de préférence entre 10 et 20 % en poids du poids du substrat de film ; et / ou
le deuxième pigment comprend du dioxyde de titane en poudre dans une quantité comprise entre 5 et 17 % en poids du substrat de film, et le matériau polymère comprend du polyvinylpyrrolidone dans une quantité comprise entre 5 et 17 % en poids du poids total du dioxyde de titane en poudre et du polyvinylpyrrolidone.

10. Le film selon l'une quelconque des revendications 1 à 4 ou le procédé selon l'une quelconque des revendications 5 à 9 dans lequel le substrat de film est présent sous forme de fragments de film, de préférence dans lequel les fragments de film ont une dimension maximale comprise entre 1 et 4 mm et une épaisseur comprise entre 0,025 to 0,075 mm.

11. Le film selon la revendication 10 dans lequel le deuxième pigment couvre sensiblement la surface entière de chaque fragment de film, de préférence dans lequel la surface entière de chaque fragment de film inclut deux faces opposées et une surface latérale périphérique entre celles-ci.

12. Le procédé selon la revendication 10 dans lequel :
les fragments de film sont formés avant que le deuxième pigment ou le deuxième pigment revêtu de matériau polymère ne soit appliqué aux fragments de film ; et / ou
dans lequel, à l'étape b1, le matériau polymère est appliqué de façon à couvrir sensiblement la surface entière de chaque fragment de film et, à l'étape c1, le deuxième pigment est appliqué de façon à couvrir sensiblement la surface entière de chaque fragment de film, de préférence dans lequel la surface entière de chaque fragment de film inclut deux faces opposées et une surface latérale périphérique entre celles-ci.

13. Le procédé selon la revendication 10, dans lequel les fragments de film sont formés après que le deuxième pigment ou le deuxième pigment revêtu de matériau polymère est appliqué au substrat.

14. Un dentifrice incluant une pluralité de fragments de film d'un film buccalement acceptable selon l'une quelconque des revendications 1 à 4 ou fabriqué par le procédé selon l'une quelconque des revendications 5 à 12.

15. Le dentifrice selon la revendication 14 comprenant entre 0,3 et 1 % en poids de fragments de film sur la base du poids du dentifrice, dans lequel :
le dentifrice est un gel translucide ou transparent ayant la pluralité de fragments de film sensiblement uniformément en suspension dans celui-ci, et / ou
le premier pigment est invisible à l'oeil nu et le deuxième pigment est visible à l'oeil nu avant le brossage des dents en utilisant le dentifrice en présence d'eau dans la cavité buccale, de préférence dans lequel le premier pigment est visible à l'oeil nu et le deuxième pigment est visible à l'oeil nu après que la matrice est sensiblement dissoute après une période de brossage des dents en utilisant le dentifrice en présence d'eau dans la cavité buccale.
